(19) Europäisches Patentamt
European Patent Office
Office européen des brevets

(11) EP 1 671 939 A1

(12) EUROPÄISCHE PATENTANMELDUNG

(43) Veröffentlichungstag:
**21.06.2006 Patentblatt 2006/25**

(51) Int Cl.:
***C07C 67/307*** *(2006.01)* ***C07B 39/00*** *(2006.01)*
***C07C 231/18*** *(2006.01)*

(21) Anmeldenummer: **05026670.9**

(22) Anmeldetag: **07.12.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **16.12.2004 DE 102004060493**

(71) Anmelder: **LANXESS Deutschland GmbH**
**51369 Leverkusen (DE)**

(72) Erfinder: **Gerlach, Arne Dr**
**65843 Sulzbach (DE)**

(54) **Verfahren zur Herstellung von enantiomerenangereicherten 2-Fluorcarbonsäureestern**

(57) Die vorliegende Erfindung betrifft ein Verfahren zur Herstellung von enantiomerenangereicherten 2-Fluorcarbonsäureestern, insbesondere (R)-2-Fluorpropionsäuremethylester, aus (S)-2-Sulfonyloxycarbonsäureestern und Kaliumfluorid.

EP 1 671 939 A1

## Beschreibung

Die vorliegende Erfindung betrifft ein Verfahren zur Herstellung von enantiomerenangereicherten 2-Fluorcarbonsäureestern, insbesondere (R)-2-Fluorpropionsäuremethylester, aus (S)-2-Sulfonyl-oxycarbonsäuresstem und Kaliumfluorid.

(R)-2-Fluorpropionsäuremethylester und andere optisch aktive 2-Fluorcarbonsäurederivate sind wichtige Synthesebausteine für die Entwicklung neuer Wirkstoffe (z.B. JP-A 2000178259) und ferroelektrischer flüssigkristalliner Phasen (z.B. DE-A 38 36 855).

In der Literatur sind mehrere Verfahren zur Herstellung von enantiomerenangereicherten 2-Fluorcarbonsäureestem, insbesondere 2-Fluorpropionsäuremethylester, beschrieben. In DE-A 41 31 242 wird beispielsweise die Herstellung von optisch aktiven 2-Fluorcarbonsäureestern aus (S)-2-Sulfonyloxycarbonsäurestern und Kaliumfluorid, insbesondere von (R)-2-Fluorpropionsäuremethylester aus (S)-2-Mesyloxypropionsäuremethylester und Kaliumfluorid in Formamid beschrieben. Hierzu wird zunächst eine Lösung von Kaliumfluorid in Formamid hergestellt und anschließend zu dieser erwärmten Kaliumfluoridlösung der (S)-2-Sulfonyloxycarbonsäurestern zugegeben. Die hier beschriebene Verfahrensweise hat aber folgende Nachteile:

1. Um die besten Ergebnisse zu erzielen, müssen beispielsweise bei der Herstellung von (R)-2-Fluorpropionsäuremethylester 4,0 Äquivalente Kaliumfluorid bezogen auf (S)-2-Mesyloxypropionsäuremethylester eingesetzt werden. Dieser große Überschuss an Kaliumfluorid ist aus industrieller Sicht nachteilig, da 3,0 Äquivalente bei dieser Reaktion ungenutzt bleiben und nach beendeter Reaktion nicht wieder gewonnen werden können.

2. Die Reaktionszeit ist mit 4 Stunden zudem relativ lang. Auch hier wäre aus industrieller Sicht eine Reduktion erstrebenswert.

3. Wird in die Reaktion (S)-2-Mesyloxypropionsäuremethylester mit einer optischen Reinheit von 97 % eingesetzt, so wird das gewünschte Produkt, der (R)-2-Fluorpropionsäuremethylester, nur mit einer optischen Reinheit von 96 % erhalten. Während der Umsetzung tritt also zu 1 % Racemisierung ein.

Es bestand somit weiterhin Bedarf an einem Verfahren zur Herstellung von enantiomerenangereicherten 2-Fluorcarbonsäureestem, insbesondere (R)-2-Fluorpropionsäuremethylester, welches diese Nachteile nicht aufweist.

Die Aufgabe der vorliegenden Erfindung bestand somit darin, ein Verfahren zur Herstellung von enantiomerenangereicherten 2-Fluorcarbonsäureestem, insbesondere von (R)-2-Fluorpropionsäuremethylester, aufzufinden, welches mit geringerem Fluoridüberschuss in kürzerer Reaktionszeit mit möglichst geringer Racemisierung zum gewünschten Produkt führt.

Überraschend wurde gefunden, dass sich enantiomerenangereicherte 2-Fluorcarbonsäureester bei der Zugabe wenigstens eines Alkalimetall- oder Tetraalkylammoniumfluorids in einem geeigneten Lösungsmittel zu vorgelegtem (S)-2-Sulfonyloxycarbonsäuresstem mit weniger als 4,0 Äquivalenten Fluorid, einer Reaktionszeit von weniger als 4 Stunden und mit einer Racemisierung deutlich unter 1% herstellen lassen.

Gegenstand der vorliegenden Erfindung ist daher ein Verfahren zur Herstellung eines enantiomerenangereicherten 2-Fluorcarbonsäureesters der allgemeinen Formel (I),

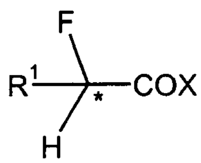

(I)

worin

$R^1$ für einen gegebenenfalls substituierten $C_1$-$C_{18}$-Alkylrest, bevorzugt für einen gegebenenfalls substituierten $C_1$-$C_6$-Alkylrest, einen gegebenenfalls substituierten $C_4$-$C_{24}$-Arylrest, bevorzugt einen gegebenenfalls substituierten $C_6$-$C_{24}$-Arylrest, oder einen gegebenenfalls substituierten $C_5$-$C_{18}$-Arylalkylrest steht und

X für Halogen, $-NR^2R^3$ oder $-OR^4$ steht, worin $R^2$, $R^3$ und $R^4$ unabhängig voneinander für H oder einen gegebenenfalls substituierten $C_1$-$C_{18}$-Alkylrest, bevorzugt für einen gegebenenfalls substituierten $C_1$-$C_6$-Alkylrest, einen gegebenenfalls substituierten $C_4$-$C_{24}$-Arylrest, bevorzugt einen gegebenenfalls substituierten $C_6$-$C_{24}$-Arylrest, oder einen gegebenenfalls substituierten $C_5$-$C_{18}$-Arylalkylrest stehen oder $R^2$ und $R^3$ zusammen für einen gegebenenfalls substituierten $C_1$-$C_{18}$-Alkylenrest, bevorzugt für einen gegebenenfalls substituierten $C_2$-$C_6$-Alkylenrest, einen gegebenenfalls substituierten $C_4$-$C_{24}$-Arylenrest, bevorzugt einen gegebenenfalls substituierten $C_6$-$C_{24}$-Arylenrest, oder einen gegebenenfalls substituierten $C_5$-$C_{18}$-Arylalkylenrest stehen,

wobei ein enantiomerenangereicherter Sulfonsäureester der allgemeinen Formel (II)

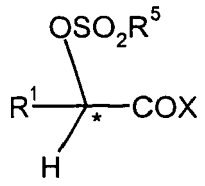

(II)

worin

$R^5$ für einen gegebenenfalls substituierten $C_1$-$C_{18}$-Alkylrest, bevorzugt für einen gegebenenfalls substituierten $C_1$-$C_6$-Alkylrest, einen gegebenenfalls substituierten $C_4$-$C_{24}$-Arylrest, bevorzugt einen gegebenenfalls substituierten $C_6$-$C_{24}$-Arylrest, oder einen gegebenenfalls substituierten $C_5$-$C_{18}$-Arylalkylrest steht und

$R^1$ und X die für die allgemeine Formel (I) genannte Bedeutung haben,

mit wenigstens einem Alkalimetall- oder Tetraalkylammoniumfluorid in einem Lösungsmittel enthaltend wenigstens ein Carbonsäureamid umgesetzt wird, dadurch gekennzeichnet, dass der Sulfonsäureester der allgemeinen Formel (II) vorgelegt und anschließend eine Mischung aus wenigstens einem Alkalimetall- oder Tetraalkylammoniumfluorid und wenigstens einem Carbonsäureamid zugegeben wird.

Bevorzugt wird der enantiomerenangereicherte Sulfonsäureester der allgemeinen Formel (II), im Folgenden auch kurz als Sulfonsäureester der allgemeinen Formel (II) bezeichnet, bei einem Druck kleiner als 1 bar, besonders bevorzugt kleiner als 500 mbar, ganz besonders bevorzugt kleiner als 50 mbar vorgelegt.

Weiterhin bevorzugt wird der Sulfonsäureester der allgemeinen Formel (II) bei einer Temperatur von mindestens 50°C, besonders bevorzugt von 50 bis 100°C, ganz besonders bevorzugt von 70 bis 85°C vorgelegt.

In bevorzugten Ausführungsformen des erfindungsgemäßen Verfahrens wird der Sulfonsäureester der allgemeinen Formel (II) bei einer Temperatur von mindestens 50°C und einem Druck kleiner als 1 bar, bevorzugt bei einer Temperatur von 50°C bis 100°C und einem Druck kleiner als 500 mbar, besonders bevorzugt bei einer Temperatur von 70 bis 85°C und einem Druck kleiner als 50 mbar vorgelegt.

Die teilweise oder vollständige Durchführung des erfindungsgemäßen Verfahrens unter Schutzgasatmosphäre, wie z.B. Stickstoff- oder Argonatmosphäre, kann von Vorteil sein, ist jedoch nicht zwingend erforderlich.

Alkyl oder Alkylen bedeutet jeweils unabhängig einen linearen, cyclischen, verzweigten oder unverzweigten Alkyl- oder Alkylen-Rest. Gleiches gilt für den nichtaromatischen Teil eines Arylalkyl- oder Arylalkylen-Restes.

**$C_1$-$C_6$-Alkyl** steht beispielsweise für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, **$C_1$-$C_{18}$-Alkyl** darüber hinaus beispielsweise für n-Heptyl und n-Octyl, Pinakolyl, Adamantyl, die isomeren Menthyle, n-Nonyl, n-Decyl, n-Dodecyl, n-Tridecyl, n-Tetradecyl, n-Hexadecyl oder n-Octadecyl.

**$C_2$-$C_6$-Alkylen** steht beispielsweise für Ethylen, n-Propylen, iso-Propylen, n-Butylen, n-Pentylen, n-Hexylen, **$C_1$-$C_{18}$-Alkylen** darüber hinaus beispielsweise für Methylen, n-Heptylen und n-Octylen, n-Nonylen, n-Decylen, n-Dodecylen, n-Tridecylen, n-Tetradecylen, n-Hexadecylen oder n-Octadecylen.

**Aryl** steht jeweils unabhängig für einen aromatischen Rest mit 4 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 24 Gerüstkohlenstoffatomen. Gleiches gilt auch für den Arylteil eines Arylalkyl- oder Arylalkylen-Restes.

Beispiele für **$C_6$-$C_{24}$-Aryl** sind Phenyl, o-, p-, m-Tolyl, Naphthyl, Phenanthrenyl, Anthracenyl oder Fluorenyl, Beispiele für heteroaromatisches **$C_4$-$C_{24}$-Aryl** in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridinyl, Oxazolyl, Benzofuranyl, Dibenzofuranyl oder Chinolinyl.

**Arylalkyl** bedeutet jeweils unabhängig einen geradkettigen, cyclischem, verzweigten oder unverzweigten Alkyl-Rest nach obiger Definition, der einfach, mehrfach oder vollständig durch ArylReste gemäß obiger Definition substituiert sein kann.

**$C_5$-$C_{18}$-Arylalkyl** steht beispielsweise für Benzyl oder (*R*)- oder (*S*)-1-Phenylethyl.

**Halogen** kann für Fluor, Chlor, Brom oder Iod, bevorzugt für Fluor, Chlor oder Brom stehen.

Das mit * gekennzeichnete Kohlenstoffatom in der allgemeinen Formel (I) oder (II) bedeutet ein asymmetrisches Kohlenstoffatom, das *(R)*- oder *(S)*-Konfiguration aufweisen kann. Bei dem erfindungsgemäßen Verfahren findet am mit * gekennzeichneten Kohlenstoffatom Konfigurationsumkehr statt.

Enantiomerenangereicherte Verbindung der allgemeinen Formel (I) oder (II) im Sinne der Erfindung

bedeutet die enantiomerenreinen Verbindungen der allgemeinen Formel (I) oder (II) in (*R*)- oder (*S*)-Konfiguration oder Mischungen der jeweiligen beiden Enantiomeren, in denen ein Enantiomer in einem Enantiomerenüberschuss, im Folgenden auch ee ("enantiomeric excess") genannt, im Vergleich zum anderen Enantiomer vorliegt. Bevorzugt beträgt dieser Enantiomerenüberschuss 2 bis 100 % ee, besonders bevorzugt mindestens 60 % ee und ganz besonders bevorzugt mindestens 85 % ee. In bevorzugten Ausführungsformen beträgt dieser Enantiomerenüberschuss mindestens 95 % ee. Eine Definition des ee-Werts wird im Rahmen der Beispiele dieser Anmeldung angegeben.

Als mögliche Substituenten für die Reste $R^1$ bis $R^5$ kommen solche Substituenten in Frage, die sich im erfindungsgemäßen Verfahren weitestgehend inert verhalten. Dies sind beispielsweise Alkoxygruppen, Carbonsäurederivate, wie Carbonsäureester, -amide, -imide, -fluoride, -chloride, -bromide und Carboxylate, Nitrilgruppen oder Fluorgruppen.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

$R^1$ steht im Rahmen der Erfindung besonders bevorzugt für einen gegebenenfalls substituierten $C_1$-$C_6$-Alkylrest, insbesondere für Methyl.

X steht im Rahmen der Erfindung besonders bevorzugt für $-OR^4$, worin $R^4$ für einen gegebenenfalls substituierten $C_1$-$C_6$-Alkylrest, insbesondere Methyl steht.

In einer bevorzugten Ausführungsform des erfindungsgemäßen Verfahrens ist der enantiomerenangereicherte 2-Fluorcarbonsäureester der allgemeinen Formel (I) (R)-2-Fluorpropionsäuremethylester.

$R^5$ steht im Rahmen der Erfindung besonders bevorzugt für einen gegebenenfalls substituierten $C_1$-$C_6$-Alkylrest oder einen gegebenenfalls substituierten $C_6$-$C_{24}$-Arylrest, insbesondere Methyl, Trifluormethyl oder p-Tolyl.

In einer bevorzugten Ausführungsform des erfindungsgemäßen Verfahrens ist der Sulfonsäureester der allgemeinen Formel (II) (S)-2-Methansulfonyloxypropionsäuremethylester.

Die Herstellung der enantiomerenangereicherten Sulfonsäureester der allgemeinen Formel (II) kann in an sich bekannter Weise durch Desaminierung der entsprechenden enantiomerenangereicherten 2-Aminocarbonsäuren mit Natriumnitrit in verdünnter Säure erfolgen. Die Desaminierung ist beispielsweise in M.Winitz et al.; J. Am. Chem. Soc. 1956, 78, 2423 beschrieben. Der in einer bevorzugten Ausführungsform als Ausgangsmaterial verwendete (S)-2-Mesyloxypropionsäuremethylester kann leicht nach Literaturvorschrift (z.B. Fritz-Langhals, E.; Schütz, G.; *Tetrahedron Letters,* **1993,** *34,* 293-296) aus kommerziell erhältlichem (S)-Milchsäuremethylester ohne Racemisierung hergestellt werden. Kommerziell erhältlicher (S)-Milchsäuremethylester hat üblicherweise eine optische Reinheit von 97 %. Damit ergibt sich für den beim Verfahren eingesetzten (S)-2-Methansulfonyloxypropionsäuremethylester ebenfalls eine optische Reinheit von 97 %.

Als Lösungsmittel werden bevorzugt ein oder mehrere Carbonsäureamide eingesetzt. Geeignete Carbonsäureamide sind beispielsweise solche der allgemeinen Formel (III)

$$R^6\text{-}C(O)NR^7R^8 \qquad (III)$$

worin

$R^6$, $R^7$ und $R^8$ unabhängig voneinander für H, einen gegebenenfalls substituierten $C_1$-$C_{18}$-Alkyl-rest, bevorzugt für einen gegebenenfalls substituierten $C_1$-$C_6$-Alkylrest, oder einen gegebenenfalls substituierten $C_4$-$C_{24}$-Arylrest, bevorzugt einen gegebenenfalls substituierten $C_6$-$C_{24}$-Arylrest stehen.

Beispielhaft für solche Carbonsäureamide seien Formamid, N-Methylformamid, N,N-Dimethylformamid, Acetamid, N-Methylacetamid oder N,N-Dimethylacetamid genannt. Bevorzugte Carbonsäureamide sind Formamid, N-Methylformamid, Acetamid oder N-Methylacetamid, besonders bevorzugt ist Formamid.

Das Lösungsmittel enthaltend wenigstens ein Carbonsäureamid kann im Rahmen der Erfindung auch weitere Lösungsmittelkomponenten enthalten, wie z.B. Wasser, Alkohole, wie Methanol, Ethanol, n-Propanol, i-Propylalkohol, Ether, wie Dioxan, Tetrahydrofuran, Diethylether, Diethylenglycoldimethylether, chlorierte aliphatische Kohlenwasserstoffe, wie Dichlormethan, Chloroform, Tetrachlorkohlenstoff, 1,2-Dichlormethan, Trichlorethylen, aliphatische oder aromatische Kohlenwasserstoffe, wie n-Pentan, n-Hexan, Hexan-isomerengemische, n-Heptan, n-Oktan, Waschbenzin, Petrolether, Benzol, Toluol, Xylole, Ketone, wie Aceton, Methylethylketon, Methyli-butylketon, Nitrobenzol oder Mischungen aus diesen. Bevorzugt enthält das Lösungmittel jedoch wenigstens ein Carbonsäureamid in einer Menge von mindestens 20 Gew.-%, besonders bevorzugt mindestens 70 Gew.-%. In bevorzugten Ausführungsformen sind im Wesentlichen keine der vorangehend aufgeführten weiteren Lösungsmittelkomponenten in dem Lösungsmittel enthaltend wenigstens ein Carbonsäureamid enthalten.

Das erfindungsgemäße Verfahren wird in Gegenwart eines Alkalimetall- oder Tetraalkylammoniumfluorids durchgeführt. Als Alkalimetall- oder Tetraalkylammoniumfluorid können im Rahmen der Erfindung auch Mischungen aus zwei oder mehreren Alkalimetall- oder Tetraalkylammoniumfluoriden verwendet werden. Besonders geeignete Alkalimetall- oder Tetraalkylammoniumfluoride sind beispielsweise Kaliumfluorid, Cäsiumfluorid oder Tetrabutylammoniumfluorid.

Bevorzugt sollte die Mischung aus wenigstens einem Alkalimetall- oder Tetraalkylammoniumfluorid und dem Lösungsmittel enthaltend wenigstens ein Carbonsäureamid mindestens 1,5 Äquivalente des Alkalimetall- oder Tetraalkylammoniumfluorids bezogen auf die vorgelegte Menge des Sulfonsäureesters der allgemeinen Formel (II) enthalten. Besonders bevorzugt sollten mindestens 1,5 aber weniger als 4.0 Äquivalente des Alkalimetall- oder Tetraalkylammoniumfluorids bezogen auf die vorgelegte Menge des Sulfonsäureesters der allgemeinen Formel (II) in der Mischung enthalten sein. In bevorzugten Ausführungsformen sind 1,5 bis 2,5 Äquivalente, in ganz bevorzugten Ausführungsformen 1,8 bis 2,5 Äquivalente des Alkalimetall- oder Tetraalkylammoniumfluorids bezogen auf die vorgelegte Menge des Sulfonsäureesters der allgemeinen Formel (II) in der Mischung enthalten.

Bevorzugt sollte die Konzentration des Alkalimetall- oder Tetraalkylammoniumfluorids in der Mischung aus wenigstens einem Alkalimetall- oder Tetraalkylammoniumfluorid und dem Lösungsmittel enthaltend wenigstens ein Carbonsäureamid mindestens 1,5 Mol pro Liter betragen.

Bevorzugt wird die Mischung aus wenigstens einem Alkalimetall- oder Tetraalkylammoniumfluorid und dem Lösungsmittel enthaltend wenigstens ein Carbonsäureamid so hergestellt, dass das oder die feste(n) Alkalimetall- oder Tetraalkylammoniumfluorid(e) in dem Lösungsmittel enthaltend wenigstens ein Carbonsäureamid vollständig oder teilweise gelöst und/oder suspendiert werden.

Die Bezeichnung Lösungsmittel bedeutet im Rahmen der Erfindung nicht, dass sich zwingend alle Reaktionskomponenten in diesem lösen müssen. Die Umsetzung kann auch in einer Suspension oder Emulsion eines oder mehrerer Reaktionspartner durchgeführt werden. Die Umsetzung kann auch in einem Lösungsmittelgemisch mit einer Mischungslücke durchgeführt werden, wobei in jeder Mischphase jeweils wenigstens ein Reaktionspartner teilweise oder vollständig löslich ist.

In einer bevorzugten Ausführungsform des erfindungsgemäßen Verfahrens wird die Mischung aus wenigstens einem Alkalimetall- oder Tetraalkylammoniumfluorid und dem Lösungsmittel enthaltend wenigstens ein Carbonsäureamid vorab auf eine Temperatur von mindestens 50°C erhitzt und dann anschließend als erwärmte Mischung zum vorgelegten Sulfonsäureesters der allgemeinen Formel (II) gegeben. Dabei kann eine portionsweise Zugabe der Mischung gegenüber einer einmaligen vollständigen Zugabe bevorzugt sein. Die portionsweise Zugabe kann innerhalb weniger Minuten bis hin zu mehreren Stunden erfolgen. In bevorzugten Ausführungsformen wird die Mischung innerhalb einer Zeit von 30 Minuten bis 4 Stunden zum vorgelegten Sulfonsäureesters der allgemeinen Formel (II) gegeben. Die Zugabe erfolgt bevorzugt bei einer Temperatur von mindestens 50°C, besonders bevorzugt von 50 bis 100°C, ganz besonders bevorzugt von 70 bis 85°C.

Nach vollständiger Zugabe der Mischung aus wenigstens einem Alkalimetall- oder Tetraalkylammoniumfluorid und dem Lösungsmittel enthaltend wenigstens ein Carbonsäureamid zum vorgelegten Sulfonsäureesters der allgemeinen Formel (II) kann es von Vorteil sein, die Reaktionsmischung noch für einen Zeitraum von einigen Minuten bis mehreren Stunden bei der gleichen Temperatur und dem gleichen Druck weiter zu rühren. Bevorzugt erfolgt dies solange, bis eine nahezu vollständige Umsetzung des vorgelegten Sulfonsäureesters der allgemeinen Formel (II) erfolgt ist. In bevorzugten Ausführungsformen wird die Reaktionsmischung mindestens 30 Minuten, bevorzugt 1 bis 5 Stunden, besonders bevorzugt 1 bis 2 Stunden bei der gleichen Temperatur und dem gleichen Druck weitergerührt, um eine möglichst hohe Ausbeute zu erzielen.

Die Mischung aus wenigstens einem Alkalimetall- oder Tetraalkylammoniumfluorid und dem Lösungsmittel enthaltend wenigstens ein Carbonsäureamid sollte vor der Zugabe zum vorgelegten Sulfonsäureesters der allgemeinen Formel (II) bevorzugt kein Wasser enthalten. Dies kann beispielsweise dadurch erreicht werden, dass unter Schutzgas die entsprechende Menge wasserfreies Fluorid in wasserfreiem Lösungsmittel gelöst wird. Es kann aber auch wasserhaltiges Fluorid und Lösungsmittel in technischer Qualität eingesetzt werden, wenn nach der Herstellung der Mischung durch Abdestillieren eines Teils des Lösungsmittels das vorhandene Wasser weitestgehend herausgeschleppt wird. Beispielsweise kann bei Verwendung von technischem Formamid als Lösungsmittel und wasserhaltigem Kaliumfluorid durch Abdestillieren von ca. 5 bis 10 % der Menge an Formamid das vorhandene Wasser weitestgehend aus der Mischung entfernt werden.

Des Weiteren kann es von Vorteil sein, wenn der gebildete enantiomerenangereicherte 2-Fluorcarbonsäureester der allgemeinen Formel (I) möglichst schnell aus dem Reaktionsgemisch entfernt wird. Dies kann beispielsweise durch kontinuierliches Abdestillieren während der Umsetzung erfolgen. Damit können sowohl Ausbeute als auch optische Reinheit des Produktes gesteigert werden. In einer bevorzugten Ausführungsform des erfindungsgemäßen Verfahrens wird der gebildete enantiomerenangereicherte 2-Fluorcarbonsäureester der allgemeinen Formel (I) während der Umsetzung bei einem Druck kleiner als 1 bar kontinuierlich in eine gekühlte Vorlage kondensiert. Besonders vorteilhaft ist hierbei eine kurze Wegstrecke zwischen Reaktionsgefäß und gekühlter Vorlage sowie eine möglichst niedrige Temperatur in dieser gekühlten Vorlage. Die gekühlte Vorlage sollte bevorzugt so angeordnet sein, dass kein Zurücklaufen des Produkts in den Reaktionsbehälter nach der Kondensation erfolgt. Die Isolierung des gebildeten enantiomerenangereicherten 2-Fluorcarbonsäureesters der allgemeinen Formel (I) kann jedoch auch erst nach beendeter Umsetzung erfolgen. Dies kann

ebenfalls durch Abdestillieren bzw. Kondensieren in eine gekühlte Vorlage erfolgen.

Der nach dem erfindungsgemäßen Verfahren hergestellte enantiomerenangereicherte 2-Fluorcarbonsäureester der allgemeinen Formel (I), insbesondere (R)-2-Fluorpropionsäuremethylester kann gegebenenfalls durch eine Feindestillation mit entsprechender Kolonne weiter aufgereinigt werden. In der Regel ist dies aber nicht notwendig, da das Produkt nach beendeter Reaktion aus der gekühlten Vorlage mit einer chemischen Reinheit von bis zu 99% isoliert werden kann.

Die folgenden Beispiele dienen der beispielhaften Erläuterung und Veranschaulichung der Erfindung, stellen jedoch keine Beschränkung dar.

**Beispiele**

In allen nachfolgenden Beispielen wird die optische Reinheit der Verbindungen der allgemeinen Formel (I) oder (II) mit (R)- bzw. (S)-Konfiguration durch chromatographische Methodem (GC oder HPLC) an einem chiralen Säulenmaterial bestimmt und über den nachfolgend definierten ee ("enantiomeric excess") (S)- oder (R)-Wert angegeben.

Der ee-Wert ergibt sich dabei über folgende Formeln:

$$ee\,(S) = \frac{m(S)-m(R)}{m\,(S+R)} \times 100\%$$

$$ee\,(R) = \frac{m(R)-m(S)}{m\,(S+R)} \times 100\%$$

wobei ee (S) bzw. ee (R) die optische Reinheit des Enantiomers S bzw. R ist, m(S) die Stoffmenge des Enantiomers S und m(R) die Stoffmenge des Enantiomers R ist. (Beispiele: Für ein Racemat gilt: R=S => ee = 0; für die reine (S)-Form gilt: ee (S) = 100 %; für ein Verhältnis von S:R = 9:1 ist ee (S) = 80 %)

**Beispiel 1:**

Von einer Mischung aus 18,59 g (0,32 mol; 2,0 Äquivalente) Kaliumfluorid und 200 ml Formamid wurden bei 110°C und 20 mbar ca. 50 ml Formamid abdestilliert. Die so erhaltene Lösung wurde dann unter Argon auf 80°C abgekühlt und in einen auf 80°C beheizten Doppelmanteltropftrichter gefüllt. Unter Argon wurden in einem 250ml Dreihalskolben 30,49 g (0,16 mol; 1,0 Äquivalent; 95,6 %ig; 97 %ee) (S)-2-Mesyloxypropionsäuremethylester vorgelegt und bei 20 mbar auf 80°C erwärmt. Anschließend wurde nun die 80°C heiße Kaliumfluoridlösung innerhalb einer Stunde zu dem (S)-2-Mesyloxypropionsäuremethylester getropft. Der entstehende (R)-2-Fluorpropionsäuremethylester wurde kontinuierlich in einer mit dem Reaktionskolben verbundenen auf -78°C gekühlten Kühlfalle aufgefangen. Nach vollständiger Zugabe der Kaliumfluoridlösung wurde noch eine weitere Stunde bei 80°C und 20 mbar nachgerührt. Aus der Kühlfalle ließen sich nach dem Auftauen 12,5 g (73% Ausbeute) (R)-2-Fluorpropionsäuremethylester mit einer chemischen Reinheit von 99 % und einer optischen Reinheit von 97 % isolieren.

**Beispiel 2:**

Von einer Mischung aus 13,94 g (0,24 mol; 1,5 Äquivalente) Kaliumfluorid und 250 ml Formamid wurden bei 110°C und 20 mbar ca. 50 ml Formamid abdestilliert. Die so erhaltene Lösung wurde dann unter Argon auf 80°C abgekühlt und in einen auf 80°C beheizten Doppelmanteltropftrichter gefüllt. Unter Argon wurden in einem 250ml Dreihalskolben 30,49 g (0,16 mol; 1,0 Äquivalent; 95,6 %ig; 97 %ee) (S)-2-Mesyloxypropionsäuremethylester vorgelegt und bei 20 mbar auf 80°C erwärmt. Anschließend wurde nun die 80°C heiße Kaliumfluoridlösung innerhalb einer Stunde zu dem (S)-2-Mesyloxypropionsäuremethylester hinzugetropft. Der entstehende (R)-2-Fluorpropionsäuremethylester wurde kontinuierlich in einer mit dem Reaktionskolben verbundenen auf -78°C gekühlten Kühlfalle aufgefangen. Nach vollständiger Zugabe der Kaliumfluoridlösung wurde noch eine weitere Stunde bei 80°C und 20 mbar nachgerührt. Aus der Kühlfalle ließen sich nach dem Auftauen 12,0 g (64 % Ausbeute) (R)-2-Fluorpropionsäuremethylester mit einer chemischen Reinheit von 90 % und einer optischen Reinheit von 97 % isolieren.

**Beispiel 3:**

Von einer Mischung aus 13,94 g (0,24 mol; 1,5 Äquivalente) Kaliumfluorid und 200 ml Formamid wurden bei 110°C und 20 mbar ca. 50 ml Formamid abdestilliert. Die so erhaltene Lösung wurde dann unter Argon auf 50°C abgekühlt und in einen auf 50°C beheizten Doppelmanteltropftrichter gefüllt. Unter Argon wurden in einem 250 ml Dreihalskolben 30,49 g (0,16 mol; 1,0 Äquivalent; 95,6 %ig; 97 %ee) (S)-2-Mesyloxypropionsäuremethylester vorgelegt und bei 20 mbar auf 50°C erwärmt. Anschließend wurde nun die 50°C heiße Kaliumfluoridlösung innerhalb einer Stunde zu dem (S)-2-Mesyloxypropionsäuremethylester hinzugetropft. Der entstehende (R)-2-Fluorpropionsäuremethylester wurde kontinuierlich in einer mit dem Reaktionskolben verbundenen auf -78°C gekühlten Kühlfalle aufgefangen. Nach vollständiger Zugabe der Kaliumfluoridlösung wurde noch eine weitere Stunde bei 50°C und 20 mbar nachgerührt. Aus der Kühlfalle ließen sich nach dem Auftauen 1,9 g (9 % Ausbeute) (R)-2-Fluorpropionsäuremethylester mit einer chemischen Reinheit von 78 % und einer optischen Reinheit von 97 % isolieren.

**Patentansprüche**

1. Verfahren zur Herstellung eines enantiomerenangereicherten 2-Fluorcarbonsäureesters der allgemeinen Formel (I),

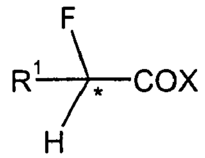

(I)

worin

$R^1$ für einen gegebenenfalls substituierten $C_1$-$C_{18}$-Alkylrest, einen gegebenenfalls substituierten $C_4$-$C_{24}$-Arylrest oder einen gegebenenfalls substituierten $C_5$-$C_{18}$-Arylalkylrest steht und
X für Halogen, $-NR^2R^3$ oder $-OR^4$ steht, worin $R^2$, $R^3$ und $R^4$ unabhängig voneinander für H oder einen gegebenenfalls substituierten $C_1$-$C_{18}$-Alkylrest, einen gegebenenfalls substituierten $C_4$-$C_{24}$-Arylrest oder einen gegebenenfalls substituierten $C_5$-$C_{18}$-Arylalkylrest stehen oder $R^2$ und $R^3$ zusammen für einen gegebenenfalls substituierten $C_1$-$C_{18}$-Alkylenrest, einen gegebenenfalls substituierten $C_4$-$C_{24}$-Arylenrest oder einen gegebenenfalls substituierten $C_5$-$C_{18}$-Arylalkylenrest stehen,
wobei ein enantiomerenangereicherter Sulfonsäureester der allgemeinen Formel (II)

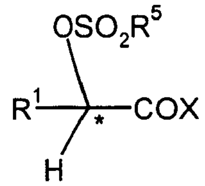

(II)

worin

$R^5$ für einen gegebenenfalls substituierten $C_1$-$C_{18}$-Alkylrest, einen gegebenenfalls substituierten $C_4$-$C_{24}$-Arylrest oder einen gegebenenfalls substituierten $C_5$-$C_{18}$-Arylalkylrest steht und
$R^1$ und X die für die allgemeine Formel (I) genannte Bedeutung haben,

mit wenigstens einem Alkalimetall- oder Tetraalkylammoniumfluorid in einem Lösungsmittel enthaltend wenigstens ein Carbonsäureamid umgesetzt wird, **dadurch gekennzeichnet, dass** der Sulfonsäureester der allgemeinen Formel (II) vorgelegt und anschließend eine Mischung aus wenigstens einem Alkalimetall- oder Tetraalkylammoniumfluorid und wenigstens einem Carbonsäureamid zugegeben wird.

2. Verfahren gemäß Anspruch 1, **dadurch gekennzeichnet, dass** der Sulfonsäureester der allgemeinen Formel (II) bei einem Druck kleiner als 1 bar vorgelegt wird.

3. Verfahren gemäß Anspruch 1 oder 2, **dadurch gekennzeichnet, dass** der Sulfonsäureester der allgemeinen Formel (II) bei einer Temperatur von mindestens 50°C vorgelegt wird.

4. Verfahren gemäß wenigstens einem der Ansprüche 1 bis 3, **dadurch gekennzeichnet, dass** $R^1$ für einen gegebenenfalls substituierten $C_1$-$C_6$-Alkylrest steht.

5. Verfahren gemäß wenigstens einem der Ansprüche 1 bis 4, **dadurch gekennzeichnet, dass** X für $-OR^4$ steht, worin $R^4$ für einen gegebenenfalls substituierten $C_1$-$C_6$-Alkylrest steht.

6. Verfahren gemäß wenigstens einem der Ansprüche 1 bis 5, **dadurch gekennzeichnet, dass** der enantiomerenangereicherte 2-Fluorcarbonsäureester der allgemeinen Formel (I) (R)-2-Fluorpropionsäuremethylester ist.

7. Verfahren gemäß wenigstens einem der Ansprüche 1 bis 6, **dadurch gekennzeichnet, dass** $R^5$ für einen gegebenenfalls substituierten $C_1$-$C_6$-Alkylrest oder einen gegebenenfalls substituierten $C_6$-$C_{24}$-Arylrest steht.

8. Verfahren gemäß wenigstens einem der Ansprüche 1 bis 7, **dadurch gekennzeichnet, dass** der Sulfonsäureester der allgemeinen Formel (II) (S)-2-Methansulfonyloxypropionsäure-methylester ist.

9. Verfahren gemäß wenigstens einem der Ansprüche 1 bis 8, **dadurch gekennzeichnet, dass** das Carbonsäureamid ausgewählt ist aus Formamid, N-Methylformamid, N,N-Dimethylformamid, Acetamid, N-Methylacetamid oder N,N-Dimethylacetamid.

10. Verfahren gemäß wenigstens einem der Ansprüche 1 bis 9, **dadurch gekennzeichnet, dass** als Alkalimetall- oder Tetraalkylammoniumfluorid Kaliumfluorid, Cäsiumfluorid oder Tetrabutylammoniumfluorid eingesetzt werden.

11. Verfahren gemäß wenigstens einem der Ansprüche 1 bis 10, **dadurch gekennzeichnet, dass** die Mischung aus wenigstens einem Alkalimetall- oder Tetraalkylammoniumfluorid und dem Lösungsmittel enthaltend wenigstens ein Carbonsäureamid mindestens 1,5 Äquivalente des Alkalimetall- oder Tetraalkylammoniumfluorids bezogen auf die vorgelegte Menge des Sulfonsäureesters der allgemeinen Formel (II) enthält.

**12.** Verfahren gemäß wenigstens einem der Ansprüche 1 bis 11, **dadurch gekennzeichnet, dass** die Mischung aus wenigstens einem Alkalimetall- oder Tetraalkylammoniumfluorid und dem Lösungsmittel enthaltend wenigstens ein Carbonsäureamid mindestens eine Konzentration von 1,5 Mol Alkalimetall- oder Tetraalkylammoniumfluorid pro Liter aufweist.

**13.** Verfahren gemäß wenigstens einem der Ansprüche 1 bis 12, **dadurch gekennzeichnet, dass** die Mischung aus wenigstens einem Alkalimetall- oder Tetraalkylammoniumfluorid und dem Lösungsmittel enthaltend wenigstens ein Carbonsäureamid auf eine Temperatur von mindestens 50°C erhitzt und dann als erwärmte Mischung zum vorgelegten Sulfonsäureesters der allgemeinen Formel (II) gegeben wird.

**14.** Verfahren gemäß wenigstens einem der Ansprüche 1 bis 13, **dadurch gekennzeichnet, dass** der gebildete enantiomerenangereicherte 2-Fluorcarbonsäureester der allgemeinen Formel (I) während der Umsetzung bei einem Druck kleiner als 1 bar kontinuierlich in eine gekühlte Vorlage kondensiert wird.

Europäisches Patentamt

# EUROPÄISCHER RECHERCHENBERICHT

Nummer der Anmeldung
EP 05 02 6670

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | E. FRITZ-LANGHALS, G. SCHUETZ: "Simple Synthesis of Optically Active 2-Fluoropropanoic acid and Analogs of High Enantiomeric Purity" TETRAHEDRON LETT., Bd. 34, Nr. 2, 1993, Seiten 293-296, XP002364902 * das ganze Dokument * ----- | 1-14 | C07C67/307<br>C07B39/00<br>C07C231/18 |
| A | E. FRITZ-LANGHALS: "Alkali Metal Fluorides as Efficient Fluorinating Agents. Enantiocontrolled Synthesis of 2-Fluoroalkyl Carboxylates and 1-Fluoroalkyl Benzenes" TETRAHEDRON: ASYMMETRY, Bd. 5, Nr. 6, 1994, Seiten 981-986, XP002364903 * das ganze Dokument * ----- | 1-14 | |
| D,A | DE 41 31 242 A1 (CONSORTIUM FUER ELEKTROCHEMISCHE INDUSTRIE GMBH, 8000 MUENCHEN, DE; CO) 1. April 1993 (1993-04-01) * Ansprüche 1-8; Beispiel 1b * ----- | 1-14 | RECHERCHIERTE SACHGEBIETE (IPC)<br>C07C<br>C07B |
| A | EP 0 749 954 A (BAYER AG) 27. Dezember 1996 (1996-12-27) * Ansprüche 1-7; Beispiel 1 * ----- | 1-5 | |

Der vorliegende Recherchenbericht wurde für alle Patentansprüche erstellt

| Recherchenort | Abschlußdatum der Recherche | Prüfer |
|---|---|---|
| Den Haag | 30. Januar 2006 | Eberhard, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................................
& : Mitglied der gleichen Patentfamilie, übereinstimmendes Dokument

2
EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 05 02 6670

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-01-2006

| Im Recherchenbericht angeführtes Patentdokument | Datum der Veröffentlichung | Mitglied(er) der Patentfamilie | Datum der Veröffentlichung |
|---|---|---|---|
| DE 4131242 A1 | 01-04-1993 | KEINE | |
| EP 0749954 A | 27-12-1996 | DE 19522703 A1 | 02-01-1997 |
| | | JP 9012508 A | 14-01-1997 |
| | | US 5723654 A | 03-03-1998 |

EPO FORM P0461

Für nähere Einzelheiten zu diesem Anhang : siehe Amtsblatt des Europäischen Patentamts, Nr.12/82